# EUROPÄISCHE PATENTANMELDUNG

(11) **EP 0 569 667 A2**
(43) Veröffentlichungstag der Anmeldung: **18.11.1993**
(21) Anmeldenummer: 93102266.9
(22) Anmeldetag: 15.02.1993
(51) Int. Cl.: A61K 7/06

(54) **Kosmetisches Haarwuchsmittel und Haarwasser gegen Haarausfall**

(30) Priorität: 13.02.1992 DE 4204255; 11.03.1992 DE 4207636; 17.04.1992 DE 4212885; 16.05.1992 DE 4216269; 03.06.1992 DE 4218209; 03.07.1992 DE 4221818; 14.10.1992 DE 4234616; 14.11.1992 DE 4238509
(71) Anmelder: Ahmad, Khalil, D-60489 Frankfurt (DE)
(72) Erfinder: Ahmad, Khalil, D-60489 Frankfurt (DE)
(74) Vertreter: Lopau, Ulrike

(57) **Zusammenfassung**

Kosmetisches Mittel zur Förderung des Haarwuchses und/oder zur Verminderung des Haarausfalls, dessen Wirkung eventuell durch gleichzeitige Verabreichung eines Arzneitees bzw. eines oralen homöopathischen Mittels gesteigert werden kann. Das Kosmetikum besteht aus einer Mischung von 45 natürlichen Substanzen in Alkohol.

## Beschreibung

Dieses Haarwuchsmittel ist sehr effektiv.

Damit benötigt man ca. 4 - 6 Monate, um wieder ausreichend Haare zu bekommen. Der Haarausfall wird in den meisten Fällen innerhalb von 2 - 5 Wochen gestoppt. Dieses System wirkt auch bei Personen, die schon lange die Haare verloren haben.

## Patentansprüche

1. Haarwuchsmittel und Haarwasser gegen Haarausfall dadurch gekennzeichnet, daß eine Mischung folgender Substanzen in 10̸0̸0̸- 160̸0̸ ml 35-90̸%igem Alkohol eingesetzt wird:
5-80̸g Apfel mit oder ohne Schale und bis 20̸g getrocknete Apfelstücke mit oder ohne Schale und bis 2g Apfelkerne, bevorzugt Sorte Red Delicious und bis zu 20̸0̸ml Apfelsaft
0̸,0̸1 - 30̸ g Sandelholz + Sandelrinde
0̸,0̸1 - 8g Koriander
0̸,0̸1 - 6g Piment
0̸,0̸1 - 6g Radies Eiszapfensamen
0̸,0̸1 - 5g Rote Rübensamen bevorzugt Sorte Rote Kugel
0̸,0̸1 - 5g Nigellasamen
0̸,0̸1 - 5g Chrysanthemumsamen
0̸,0̸1 - 6g Malvenblüten oder Malvensamen bevorzugt Sorte Malva Sylvestris
2 - 80̸g Maracuja Frucht mit oder ohne Schale und bis 10̸g getrocknete Maracuja Frucht mit oder ohne Schale und bis 5g Maracujasamen/Passiflorasamen und bis 40̸ml Maracujasaft und bis 4g Maracujakraut
1 - 30̸0̸ Tropfen Thuja Tinktur
0̸,0̸0̸1 - 4g Mangold-Lukullussamen oder Pflanze
1 - 90̸0̸ Tropfen der Mischung von Sepia Gruneris und 50̸%igem Alkohol, wobei der Gehalt von Sepia Gruneris 15% ist und der von Alkohol 85%
0̸,0̸1 - 5g Rosmarinblätter/Rosmarinkraut
0̸,0̸1 - 5g Schafgarbeblätter/Schafgarbekraut
0̸,0̸1 - 40̸ml Birkenblättersaft oder bis 10̸g Birkenblätter
0̸,0̸1 - 26ml Johanniskrautsaft und bis zu 6g Johanniskraut
0̸,0̸1 - 4g Pfefferminzblätter
0̸,0̸1 - 6g Brennesselblätter
0̸,0̸1 - 4g Bockshornkleesamen
0̸,0̸1 - 4g Gelbe Senfkörner
0̸,0̸1 - 4g Kohlrabisamen bevorzugt Sorte Delikates Blauer
0̸,0̸1 - 4g Rosenblüten
0̸,0̸1 - 2g Klettenwurzel
0̸,0̸1 - 3g Wacholderbeeren
0̸,0̸1 - 4g Arnikablüten
0̸,0̸1 - 3g Oliven
0̸,0̸1 - 3og Baldrianwurzel oder sonstige Baldrianpflanzenteile
0̸,1 - 70̸ml Traubensaft und bis zu 4g Traubenkerne
0̸,0̸1 - 30̸ml Orangenblütensaft und bis 8g Orangenblüten und bis 4g Orangenschale
0̸,0̸1 - 3g geschälte Honigmelone oder Honigmelonensamenkerne
0̸,0̸1 - 3g Kokosnuß
0̸,1 - 30̸ml Pfirsichsaft und bis 5g Pfirsichblüten und bis 20̸g Pfirsichfrucht mit oder ohne schale/Haut und bis20̸g getrocknete Pfirsichfrucht mit oder ohne Schale/Haut
0̸,1 - 150̸ml Kiwisaft und bis 4g Kiwisamen/Kerne und bis 80̸g Kiwifrucht mit oder ohne Schale und bis 20̸g getrocknete Kiwifrucht mit oder ohne Schale
0̸,0̸1 - 5g Krautmelisse
0̸,1 - 150̸ml Granatapfelsaft und bis 4g Granatapfelkerne und bis 15g Granatapfelschale + bis 10̸% innere Häute
0̸,0̸1 - 5g Jojobablätter und/oder Jojobasamen
0̸,0̸1 - 5g Linsenurid (geschält)
0̸,0̸1 - 3g Süße Mandel ohne Schale
0̸,0̸1 - 4g Coffeasamen (Kaffeebaumsamen) oder bis 4g Kaffee
0̸,0̸1 - 5g Preiselbeerblätter
0̸,0̸1 - 5g Frauenhaarblätter
0̸,0̸1 - 10̸0̸ml Kalium Phosphoricum D2 - D 5(homöopathisches Mittel)
0̸,0̸1 - 10̸0̸ml Azadirachta Indica D1 (homöopathisches Mittel)
0̸,0̸1 - 10̸0̸ml Jaborandi D 1 (homöopathisches Mittel)
0̸,1 - 10̸0̸ml Birnensaft und bis 80̸g Birnenfrucht mit oder ohne Schale/Haut und bis 30̸g getrocknete Birnenfrucht mit oder ohne Schale/Haut und bis 3g Birnenfruchtkerne

2. Nach Anspruch 2 wird dieses Haarwasser/Haarwuchsmittel in 2 Flaschen gegeben. Eine Flasche wird mit der kompletten Formel hergestellt und eine Flasche wird ohne Sepia Gruneris hergestellt. Das Haarwasser/Haarwuchsmittel ohne Sepia Gruneris ist regelmäßig benutzbar. Das Haarwasser/Haarwuchsmittel mit Sepia Gruneris wird einmal alle 8 - 60̸ Tage benutzt.

3. Nach Anspruch 3 werden die Säfte einfach ins Haarwasser/Haarwuchsmittel gemischt und dazu der Alkohol gegeben.

4. Das Endprodukt muß 25 - 60̸% Alkohol enthalten.

5. Homöopathisches Hilfsmittel für neuen Haarwuchs und gegen Haarausfall in folgender Kombination (D = homöäopathisches Potenzzeichen und ϑ = Urtinktur)
D6 - D60̸ Indigo
D2 - D60̸ Kalium Phosphoricum
D1 - D60̸ Granatum
D1 - D60̸ Azadirachta Indica
D1 - D60̸ Jaborandi
ϑ - D60̸ Nigellasamen
ϑ - D60̸ Chrysanthemumsamen
ϑ - D60̸ Mangold-Lukullussamen
ϑ - D60̸ Maracujasamen (Passiflorasamen alle Sorten)
ϑ - D60̸ Sandelholz/Sandelrinde
ϑ - D60̸ Rote Rübensamen (bevorzugt Rote Kugel)
ϑ - D60̸ Malvenblüten (bevorzugt Malva Sylvestris)
ϑ - D60̸ Melissenkraut
ϑ - D60̸ Piment
ϑ - D60̸ Kiwisamen/Kiwikerne

6. Um von den letzten 10̸ Substanzen bei Anspruch 5 die Urtinktur herzustellen, werden die Wirkstoffe/Extrakte jeweils aus bis zu 1,5 g Grundsubstanz in 9 ml 35-90̸%igem Alkohol gemischt/gezogen. Ab D 2 wird Alkohol Vol 35-56% benutzt.

7. Um die Wirksamkeit des Haarwuchsmittels/Haarwasser gegen Haarausfall zu steigern, wird Tee aus folgenden Kombinationen getrunken. (Wassermenge für jede Tasse ca 185 ml):
(1) 0̸,0̸0̸1 - 0̸,30̸ g Mangold-Lukulussamen +0̸,20̸ -15g getrocknete Apfelstücke + bis 20̸g getrocknete Birnenstücke
(2) 0̸,2 - 3 g Kiwifrucht (getrocknet) + bis 2 g Orangenblüten + bis 0̸,5 g Traubenkerne
(3) bis 4 g Maracuja Frucht (getrocknet) + bis 1 g Malvenblüten + bis 1 g Granatapfelkerne

8. Die in Anspruch 7 genannten Kombinationen können auch als wasserlösliche Tabletten aus Extrakten und Lactose hergestellt werden. Eine Tablette kann bis zu 3 g wiegen,wobei der Anteil von Extrakten bis zu 40̸% betragen kann. Diese 3 Kombinationen können auch in angegebenen Werten weiter miteinander kombiniert und auch in Tablettenform hergestellt werden. Diese Tabletten werden in heißem Wasser aufgelöst und als Tee getrunken.
